# EUROPEAN PATENT SPECIFICATION

(11) **EP 2 231 267 B1**
(45) Date of publication and mention of the grant of the patent: **18.12.2013**
(21) Application number: 08864282.2
(22) Date of filing: 19.12.2008
(51) Int. Cl.: A61N 1/36

(54) **TREATMENT OF PELVIC DISORDERS BY ELECTRICAL NERVE STIMULATION**
BEHANDLUNG VON BECKENERKRANKUNGEN DURCH ELEKTRISCHE NERVENSTIMULATION
TRAITEMENT DE TROUBLES PELVIENS PAR NEUROSTIMULATION ÉLECTRIQUE

(30) Priority: 21.12.2007 EP 07150387; 21.12.2007 US 8587 P
(43) Date of publication of application: 29.09.2010
(73) Proprietor: Neurodan A/S, 9200 Aalborg SV (DK)
(72) Inventor: FJORBACK, Morten, DK-9260 Gistrup (DK); RIJKHOFF, Nico J. M., DK-9210 Aalborg Sø (DK); BORUP, Thomas, DK-9530 Støvring (DK); ERIKSEN, Michael, DK-9240 Nibe (DK)
(74) Representative: Inspicos A/S
(86) International application number: PCT/EP2008/068096
(87) International publication number: WO 2009/080785

(56) References cited:
- WO-A2-2007/075974
- US-A1- 2005 015 119
- US-A1- 2006 122 660
- US-A1- 2007 049 991
- US-A1- 2007 060 967
- US-A1- 2007 255 333

## Description

### Technical field

The present invention is generally concerned with the treatment of urge incontinence by electrical stimulation of pudendal nerve afferents, such as dorsal penile/clitoral nerves.

### Background of the invention

Electrical stimulation of pudendal nerve afferents is known to be effective in the treatment of a variety of pelvic disorders, such as urge urinary incontinence. The symptoms of increased daytime frequency, nocturia and urge urinary incontinence are often related to detrusor overactivity (DO), which is characterized by involuntary bladder contractions at low bladder volumes. The aforementioned symptoms adversely affect patients' quality of life due to social and hygienic difficulties. Upper urinary tract damage caused by sustained high intravesical pressures and repeated bladder infections is another concern that causes morbidity, hospitalization or even death in patients with DO. Bladder dysfunction is usually one of the most challenging long-term management issues in neurological patients because of the limited treatment options to help relieve symptoms. Approximately 1 million patients with symptoms of an overactive bladder (35% urge incontinence) are refractory to conventional treatment options world-wide. Especially neurological patients suffering from e.g. multiple sclerosis (MS), spinal cord injury (SCI), or stroke (CVA) are often difficult to treat by conventional means.

One way of electrically stimulating nerves is to convey electrical pulses to the nerves via an electrode implanted at or near a nerve or nerve afferent. Pulses are normally generated by implanted or external pulse generators, from which the electrical pulses are transmitted via an implanted or a transcutaneous lead.

Some of the known systems suffer from the disadvantage that the patient may feel discomfort when pulse stimulation is initiated due to the sudden application of electrical currents to the patient's nerves or nerve afferents.

US-A1-2007/060967 discloses a system according to the pre-amble of claim 1. One disadvantage of this system is that it continuously consumes a relatively high amount of power.

### Summary of the invention

It is an object of embodiments of the present invention to provide a system for electrical nerve stimulation, in which power consumption is reduced. It is a further object of embodiments of the present invention to provide a system and a method which reduce patients' sensation of discomfort upon activation of electrical stimulation of pudendal nerve afferents for the treatment of urge incontinence. It is a further object of embodiments of the invention to provide a system and method, in which power consumption can be minimized.

The invention provides a system according to claim 1.

In the present invention, power consumption is reduced by the system entering the dormant mode. The dormant mode of this invention is to be understood as the time period between the event-triggered stimulation puls(es) has/have been sent from the pulse generator and the next wake up signal has been received by the pulse generator. Hence the duration of the dormant mode may vary during the day. In the dormant mode the stimulator's stimulation circuits consume very little or no power at all. Only a circuit for wakening up the entire stimulator consumes power. In case the system relies on wireless inductive telemetry for transmitting data to the implanted pulse generator, energy for initiating wake-up of the pulse generator may be transmitted from an external device, i.e. by inductive energy transfer. In such embodiments, the power consumption of the wake-up circuit is reduced to a minimum.

In the present context, the term 'dormant mode' is hence not a mode, in which the pulse generator is completely disabled. To the contrary, the pulse generator is in a standby state, in which it is capable of receiving a signal transmitted from an external device for causing the pulse generator to initiate the generation of a train of pulses. Thus, in this invention the dormant mode occurs several times during the day, i.e. following each and every time a pulse train has been delivered. The dormant mode accordingly occurs in cycles, the duration and frequency of which depend from the duration and frequency of user activation of the generation of pulses.

In the dormant mode, the pulse generator as such consumes no power. A wake-up circuit is capable of causing the pulse generator to exit the dormant mode and enter the active mode in response to the control signal. In one embodiment, the system according to the present invention hence comprises:
- a wake-up circuit for providing said control signal to the pulse generator to cause it to exit the dormant mode and enter the active mode;
- at least one power source for powering the pulse generator; wherein the power source is configured such that no voltage is maintained at the pulse generator in the dormant mode.

The wake-up circuit may be entirely housed or integrated in the pulse generator, or it may be provided as a separate implantable or external component. The power source may comprise one or more batteries, included e.g. in a housing of the pulse generator. Alternatively, the power source may include an external power source for transmitting power to the pulse generator through wireless inductive transmission or by means of a percutaneous wire. In one embodiment, the system comprises an external or implantable power source for powering the pulse generator, so as to reduce the frequency of battery exchange and/or battery recharge.

The wake-up signal transmitted to the implantable pulse generator may include stimulation parameters, such as pulse voltage or current amplitude, pulse shape, pulse duration or frequency. Alternatively, these parameters may be provided immediately following the wake-up signal. In a still further alternative, the parameters may be pre-stored in a memory of the pulse generator. If a plurality of stimulation programs are pre-stored in the pulse generator, the external device may transmit a program selection along with the wake-up signal or immediately following the wake-up signal, allowing the pulse generator to choose the desired stimulation program.

The signals provided by the external device may be provided through a percutaneous wire or as a wireless signal, e.g. an inductive signal, preferably of a frequency below 315 kHz, such as in the ULP-AMI band of 9 - 315 kHz according to the TR 101 981 or EN 302 195 standards, or a radio frequency signal, preferably of a frequency above 400 MHz, such as in the range of the ULP-AMI band of 401-406 MHz, or in the MICS band of 402-405 MHz, both according to the TR 102 343 standard, or according to the EN 301 839 standard (402-405 MHz).

Generally, for the purpose of charging a battery within the implantable pulse generator, wireless inductive signals should be provided at a frequency of below 315 kHz or below 300 kHz, preferably around 125 kHz. Inductive charging avoids battery replacement by surgical intervention.

In the present context, electrodes are said to be placed or placeable at, on, in, near or around nerves. It should be understood that any reference to the placement of electrodes is not limited to any specific of these locations. Hence, an electrode, which for example is said to be placed or placeable 'at' a nerve may be placed or placeable not only 'at', but also on, in, near or around that nerve, unless the present text specifically requires a certain location of the electrode. In general, the reference to any placement'on', 'in', 'near' or 'around' nerves includes any placement 'at', 'on', 'in', 'near' or 'around'.

As used herein, the reference to pudendal nerve afferents includes the sensory part of the sacral nerves, dorsal penile/clitoral nerves (genital nerves), as well as any parts or branches thereof.

In one embodiment, the pulse generator is further configured to, subsequent to having entered the active mode, stepwise increase charges of electrical pulses from an initial level to a final level, and to subsequently provide the electrical stimulation pulses at said final level for a predetermined period of time. It will hence be appreciated that the electrical stimulation pulses are initially provided at a relatively low charge, so as to reduce the patient's sensation of discomfort in comparison to prior art systems and methods, which from the outset provide stimulation at the final charge level. The initial charge level is preferably zero. The gradual increase of the pulse charges may lead to higher patient comfort, not only during stimulation start-up, but also during the entire stimulation period.

A stepwise decrease of pulse charges from the final level back to the initial level (e.g. zero) following stimulation is also possible.

The control signal to the pulse generator is preferably provided or initiated by the patient, thereby allowing for event-triggered activation of stimulation in contrast to prior art systems relying on continuous stimulation. The problem of patients' sensation of discomfort is of particular relevance in an event-triggered setup, because stimulation is initiated frequently, commonly several times a day. Stimulation is normally carried out less than five times per hour, and each stimulation period typically lasts from about 30 seconds to about several minutes, such as 1, 2 or 5 minutes. Consequently, the system is inactive for at least 50% of the time, typically for at least 95% of the time. The provision of a dormant mode, in which the system consumes no power or only a minimum amount of power is therefore particularly beneficial in an event-triggered system. In the present context, the term 'dormant mode' is to be understood as a mode, in which a voltage is maintained at the wake-up circuit.

The stepwise increase of pulse charges (ramp up) may be provided as a user-selectable option. Hence, the user may for example select various stimulation programs, of which some provide stepwise increase of pulse charge, and some provide immediate stimulation at the final level without prior stepwise increase of the pulse charges. Likewise, the parameters of the stepwise pulse increase may be set according to a user selection. For example, the user may be allowed to choose ramp-up time, frequency, number of pulses to be provided during ramp-up etc.

In order to allow user-initiated triggering, the system of the present invention may comprise a user-operable triggering mechanism configured to transmit a control signal to the implantable pulse generator in order to trigger the sequence of electrical pulses. The triggering mechanism may conveniently comprise a user-interface configured to allow the user to set stimulation parameters.

In some setups the system is programmed to provide a train of a predetermined number of pulses or to calculate the number of pulses (i.e. the duration of the train of pulses), whereby the train of pulses is determined by the control of the system, unless overruled by a patient-activated emergency stop. In other setups, it may however be desirable to allow the patient to prolong the stimulation period, whereby the system is programmed to generate electrical pulses until the patient stops the pulse generation. Prolonged stimulation periods may in particular be useful for extending the patient's continence time immediately prior to bladder voiding, usually for a period of time of 1-5 minutes prior to voiding. User-activated termination of the train of pulses may also be beneficial in cases the patient reaches a toilet facility before expiration of a predetermined or pre-programmed train of pulses, so as to expedite bladder voiding. The possibility of user-activated termination of the pulse train also constitutes a safety feature, which may reduce the risk of nerve damage due to excessive stimulation and/or reduce the patient's sensation of discomfort.

The level of charge of the electrical pulses may be controlled by controlling one or more of:
- the amplitude of current of the electrical stimulation pulses;
- the amplitude of voltage of the electrical stimulation pulses;
- the pulse rate of the electrical stimulation pulses, i.e. the length of intervals between the pulses;
- the pulse width of the electrical stimulation pulses;
- the shape of the electrical stimulation pulses, however square pulses are preferred.

The pulse generator may be configured to stepwise increase the pulse charges from the initial level to the final level during a period of time of about 0.1 seconds to about 40 seconds. The stepwise increase of the pulse charges may be linearly or exponentially increased.

The pulse generator is preferably configured to provide the pulses at a pulse rate of about 5 pulses per second to about 50 pulses per second. In preferred embodiments, the initial level provides no current, whereas the final level provides charges at a current from about 0.01 mA to about 100 mA, such as from about 0.01 mA to about 10 or 20 mA. The at least one electrode is preferably configured to be placed at, on, in, near or around a genital nerve, such as a dorsal penile/clitoral nerve.

The pulses are typically provided at a pulse width of about 50 µs to about 400 µs, such as at a pulse width of about 200 µs.

The at least one electrode is implanted or implantable at a left and/or right branch of the dorsal penile/clitoral nerve, which is a distal portion of the pudendal nerve close to the genitals. The electrode may be implanted at or near the genitals or in Alcock's Canal via the perineum.

The pulse generator may be implantable/implanted in a subcutaneous pocket in the pelvic or abdominal region at a tissue depth of approximately 4 cm or less. In other embodiments, the pulse generator may be implantable/implanted to a tissue depth of between 0.3 cm and 0.4 cm or between 0.6 cm and 1.4 cm, or between 1.6 cm and 1.9 cm or between 2.1 and 2.8 cm. The pulse generator is preferably implanted in a subcutaneous pocket in the pelvic or abdominal region, such as in the anterior pelvic or abdominal region. The implant may contain means for fixation, e.g. sutures.

Preferably a wireless control signal may be transmitted to the implanted pulse generator from a position outside the living being's body in order to trigger a sequence of the electrical pulses, the control signal comprising an inductive signal. An implanted/implantable battery may advantageously be provided in order to power the pulse generator. Thanks to the provision of the implantable battery, the only inductive signal that is required for the operation of the system of the present invention is a triggering signal. Once the pulse generator has been triggered, the power for the pulses is provided by the implantable battery. Hence, the triggering mechanism need only be placed in the vicinity of the pulse generator during the period of triggering the pulse generator. The triggering period is comparatively very short compared to the stimulation period. In essence, triggering merely requires an activation signal which is transmittable in less than 1 second, whereas the stimulation period typically lasts for a predetermined period of approximately 1-2 minutes, unless stimulation is performed continuously, i.e. during periods of several hours.

The battery is preferably hermetically sealed within an implanted housing. For example, the battery may be provided in hermetically sealed compartment of a housing of the pulse generator.

Moreover, due to the provision of an inductive triggering signal there is no requirement of a continuously listening receiver, and consequently less power is consumed. This in turn extends the life time of the implantable battery or, alternatively, allows for a smaller battery to implanted.

In particular, the pudendal nerve branches, such as the genital nerves (penis nerve in men and clitoris nerve in women) may be stimulated by the system and method of the present invention. The invention is particularly useful for the treatment of urge urinary incontinence, but may also be applied in the treatment of faecal incontinence. Treatment of stress incontinence is also contemplated. Further, the invention may be useful for bladder voiding by stimulation of the perineal nerve afferents.

As outlined above, the system of the present invention is preferably event-triggered. In other words, stimulation may be initiated by a patient, once the patient feels a compelling desire to pass urine or faeces. The method of the present invention preferably provides direct sensory nerve stimulation to evoke a detrusor/bowel inhibitory reflex together with reflex mediated contraction of pelvic floor muscles that aids to preserve continence. In respect of applications, in which the electrode is implanted on, at or near a dorsal penile/clitoral nerve, stimulation only affects pudendal nerve afferents, i.e. stimulation is selectively targeted towards nerve afferents that reflexively affect the bladder. This is fundamentally different from systems, which provide continuous neuromodulation to modulate the spinal reflex arches, without allowing the patient to initiate stimulation. In such systems, urge to empty the bladder or bowel is suppressed for a period of time, however once the patient feels urgency, such urgency cannot be suppressed by the stimulation. Hence, no warning is given to the patient, who may therefore not have sufficient time to reach a toilet. The present invention provides a system that allows for patient-initiated stimulation. The patient will typically initiate stimulation once he or she feels a desire to empty the bladder or bowel, whereby the stimulation causes the urge to vanish for a period of time of e.g. 10 minutes, allowing the patient sufficient time to reach a toilet.

Though it is preferable that the patient may initiate stimulation for the above reasons, the systems and methods of the present invention may be configured to provide continuous stimulation under certain circumstances, e.g. when the patient is asleep. Alternatively, if the patient's is physically capable of preserving continence when he or she is asleep, the system may be inactivated when the patient is asleep.

The at least one implantable electrode may include a cuff electrode, a wire, a helix, a double helix, a spiral electrode, or an intra-neural electrode. Preferably, the electrode is configured to be placed in the immediate vicinity of a pudendal nerve branch or around a nerve branch.

The pulse generator includes a receiver for receiving inductively transmitted signals emitted by the triggering device. Preferably, the receiver is configured to receive inductive signals only, so that it requires no power supply. The pulse generator may further include an electronic memory for storing stimulation programs and/or stimulation parameters. The electronic memory is preferably provided as a non-power consuming memory, such as EPROM, EEPROM, flash memory or the like known per se.

The triggering mechanism may be user-controlled to allow the user to initiate pulse stimulation. Hence, stimulation may be event-triggered, e.g. following a compelling desire to pass urine or faeces. The triggering mechanism is preferably provided as an external (i.e. non-implanted) device, which may include a programmable memory for storing stimulation programs and/or stimulation parameters.

In preferred embodiments, the lead is detachably connected to the pulse generator, e.g. through a releasable connector. The lead may be permanently secured to the electrode or detachably coupled thereto.

In one embodiment, the wireless control signal is transmittable through a body portion of the living being within a distance of at most 2.5 m, such as most 1.5 m, 1 m or at most 50 cm from the pulse generator. Thereby, the risk of false or unintended triggering is reduced, and the risk of interference of the surrounding environment is reduced. The pulse generator may be configured such that it is unable to detect the inductive triggering signal if the triggering mechanism is at a distance of more than 2.5 m away from the pulse generator. Alternatively or additionally, the triggering mechanism may be configured such that the emitted inductive signals are sufficiently weak in order for them to be undetectable at a distance of more than 2.5 m from the triggering mechanism.

As the triggering mechanism is external (i.e. non-implanted) the inductive triggering signal has to travel through atmospheric air, clothing and body tissue. The above reach limitation of approximately 2.5 m is to be understood as a reach through atmospheric air.

In one embodiment the battery is rechargeable. For example, the battery may be rechargeable while it is left in place in the patient's body. In one embodiment the battery is inductively rechargeable, e.g. by means of the triggering device and a combined trigger and power receiver of the implanted pulse generator. Alternatively, the battery may be removed by surgical intervention and recharged outside the patient's body. In other embodiments, the battery is non-rechargeable. In such cases the battery life time may exceed the expectation of the patient's remaining life time or battery replacement may be performed by surgical intervention.

The pulse generator may be configured to provide the electrical pulses when it is in an active mode and to enter a dormant mode upon completion of a sequence of the pulses. The user-operable triggering mechanism may be configured to provide the inductive control signal with an amount of energy sufficient to cause the pulse generator to switch from the dormant mode to said active mode. By causing the pulse generator to enter the dormant mode, power consumption may be kept at a minimum level, in particular as the power required to cause the pulse generator to switch from the dormant mode to the active mode is provided by the triggering mechanism.

The triggering mechanism may be configured to include at least one of the following commands in the control signal:
- a wake-up command for the implantable pulse generator;
- a command for the pulse generator to select a particular one of a plurality of stimulation programs pre-stored in a memory of the implantable pulse generator; and
- stimulation parameters of said stimulation pulses, including at least one of: an amplitude of the electrical pulses; a voltage of the electrical pulses; a pulse width of the electrical pulses; a pulse rate of the electrical pulses.

In order to avoid tissue damage, the implantable pulse generator may be configured to control stimulation parameters of the stimulation pulses and to charge balance the pulses. Charge balancing may be achieved by alternating polarity, by shortcircuiting the electrodes after each stimulation pulse, through a capacitor coupled in series with the electrodes, or by software-controlled means.

In order to allow the implanted pulse generator to communicate to external device, the triggering mechanism may comprise a signal receiver for receiving a wireless signal from the implantable pulse generator. The implantable pulse generator may in such an embodiment be configured to transmit a wireless signal to the triggering mechanism, e.g. in case of one or more predetermined conditions of the implantable components, such as upon detection of a low battery charge. In one embodiment, the pulse generator is configured only to communicate a low battery status. In other embodiments, other information may be transmitted by the implanted pulse generator, such as information about malfunctions in the implanted components.

The triggering mechanism may be included in a portable device, for example in a device which is sized and configured to be worn around the wrist of a human being. For example, a wrist watch may be integrated with the triggering mechanism. Other portable devices are possible, e.g. devices which may be worn in a necklace, belt clip, devices integrated in cellular telephones or in other common personal items.

The triggering mechanism and the implanted pulse generator may be configured such that the wireless control signal and/or wireless triggering signal is only receivable by the pulse generator when the wrist-worn triggering mechanism is within a predetermined range from the implanted pulse generator, e.g within a distance of 2.5 m or less.

The triggering mechanism may be configured to wirelessly transmit stimulation parameters to the implantable pulse generator. Such stimulation parameters may include at least one of a current or voltage amplitude of the electrical pulses, a shape of the electrical pulses, a pulse width of the electrical pulses, and a frequency of the electrical pulses.

For patient convenience, the triggering mechanism comprises a user interface configured to emit a signal indicative of at least one of:
- a position of the user and/or of a public toilet facility and/or a distance to the nearest public toilet facility;
- time elapsed since last bladder voiding and/or since last stimulation pulse train;
- time, alarm and/or date;
- log data related to past stimulation pulse trains;
- log data related to past voiding, such as volume of voided urine.

Triggering of the pulse generation may in embodiments of the present invention be performed in response to a signal provided by a sensor for determining the bladder pressure or volume. In such embodiments, the triggering mechanism can be dispensed with, but may be provided as back-up device allowing the user to trigger pulse generation.

An external programming device may be provided in order to transmit the pulse stimulation parameters or pulse generation programs to the implantable pulse generator. The external programming device may be at the disposal of the patient, or it may alternatively be provided at a medical facility, operable by a trained physician only. Preferably, the external programming device includes suitable software, user interface, memory, power unit, and data processor.

The system of the present invention may comprise an external programming device for transmitting data to the pulse generator when implanted, the external programming device and the pulse generator being configured to transmit and receive said data via wireless radio communication or inductive telemetry. Generally, radio frequency signals have a wider range than inductive signals, and for certain purposes it may hence be beneficial with respect to user and programmer convenience that programming and other data transmission to the pulse generator is carried out by means of radio frequency signals.

In one embodiment, the user-operable triggering mechanism comprises a skin-borne device for transmitting inductive wireless control signal to the implantable pulse generator, and a user activatable device for transmitting a wireless radio frequency signal to the skin-borne device upon user activation of the user activatable device. The skin-borne device is preferably configured to receive the wireless radio frequency signal transmitted from the user activatable device and to generate the inductive wireless control signal for the implantable pulse generator in response thereto. Hence, the skin-borne device may be attached to a body portion of the user in the vicinity of the implantation site of the pulse generator, e.g. at the abdominal wall, whereas the user activatable device may be at a distance therefrom, e.g. around the user's wrist, integrated in a mobile phone, PDA, or provided in a separate hand-held device. Hence, in comparison to embodiment, in which the inductive wireless signal is provided directly from a user-held device to the pulse generator, the present device confers the benefit that the user does not need to observe the limited range of inductive wireless transmission.

A non-implanted (i.e. external) battery may be provided for powering the skin-borne device, e.g. within the skin-borne device itself.

An implantable battery may be provided for powering the pulse generator. However, as described herein the pulse generator and possibly other implantable parts may be powered solely by inductive power transmission from an external, non-implanted device, such as the skin-borne device.

The lead connecting the pulse generator to the at least one electrode may be helical in order to provide a flexible and mechanically strong connection. The lead may be coated with a biocompatible material or made entirely from a biocompatible material.

The housing of the pulse generator is preferably coated with or made from a biocompatible material, such as titanium.

As an alternative to inductive, wireless control signals, the system of the invention may be rely on an implantable user-operable triggering mechanism configured to transmit the control signal to the implantable pulse generator, the implantable user-operable triggering mechanism being configured to transmit said control signal in response to a mechanical impact to an outer surface portion of the user's body.

Due to the provision of a mechanical triggering signal there is no requriement of a continuously listening receiver, and consequently less power is consumed. The implantable user-operable triggering mechanism, which is operable by a mechanical impact to an outer surface portion of the patient's body, eliminates the need for an external triggering device to be worn or carried by the patient. Hence, the patient's handling of the system is facilitated, as those parts, which are required in the case of a need for immediate operation of the stimulation system, are all implanted/implantable.

The implanted/implantable triggering mechanism may be configured to be activated in response to various types of mechanical impacts, such as pressure or acceleration, pressures/sound waves. Hence, the triggering mechanism may include at least one of: a pressure switch/pressure sensor, an accelerometer, and a pressure transducer.

In embodiments including a mechanical triggering mechanism, the implanted/implantable battery may be provided as a separate, implantable component, or it may be provided within a housing of the triggering mechanism or within a housing of the pulse generator. Separate batteries may be provided for the triggering mechanism and the pulse generator, respectively, may be provided. The battery or batteries provide power for the control signal transmitted by the triggering mechanism, for the pulse generator and optionally also for the wireless transmission of signals from the implanted components to an external device, e.g. a control device.

Embodiments including a mechanical triggering mechanism may conveniently be combined with pulse generators capabable of entering a dormant mode, in which the pulse generator as such consumes no power. In the dormant mode, there is no need to maintain a voltage at any circuit, as the mechanical triggering mechanism may close a circuit providing the required wake-up current to the pulse generator. However, in other embodiments, it may be desirable to maintain a voltage at a circuit of the pulse generator, in order to achieve fast wake up of the generator or in order to allow the pulse generator to perform certain functions in the dormant mode, e.g. low-battery check, hardware function tests etc.

### Description of the drawings

Embodiments of the invention will be described with reference to the accompanying drawings, in which:
Fig. 1 shows an illustration of the pelvic region of a patient, including an implanted electrode, pulse generator and lead, as well as an external triggering mechanism;
Fig. 2 shows an illustration of the pulse generator shown in Fig. 1;
Figs. 3 and 4 are graphical representations of biphasic stimulus current outputs from the pulse generator;
Figs. 5 and 6 are illustrations of a wireless external programming device;
Fig. 7 is an anatomic view showing the implanted pulse generator of Figs. 1 and 2 in association with a clinical programming system;
Figs. 8 and 9 are illustrations of an implanted triggering mechanism, which is activatable by mechanical impact to an outer surface portion of the patient's body;
Figs. 10-12 are flow charts of various processes of the system of the present invention;
Fig. 13 is an illustration of a sterilized kit including implantable elements as well as surgical tools for their implantation;
Fig. 14 illustrates a releasable connection between a pulse generator and the lead connecting the pulse generator to the electrode;
Fig. 15 shows a further embodiment of a system according to the invention.

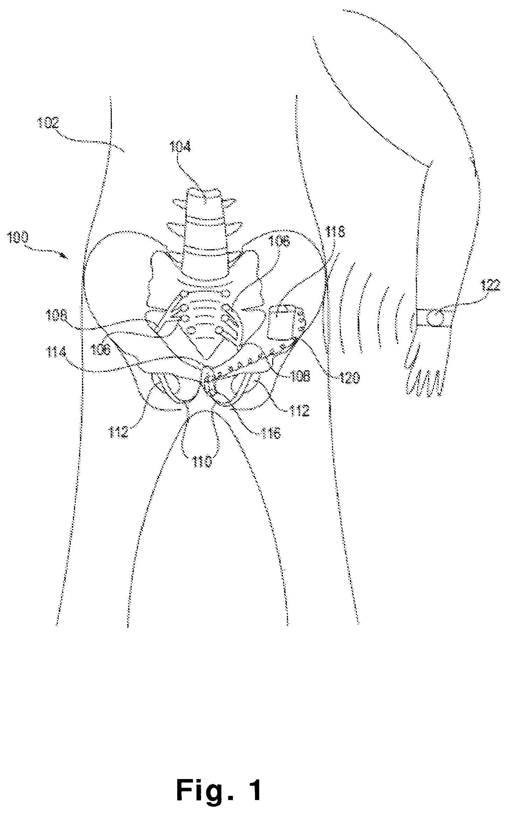

Fig. 1 generally illustrates the pelvis 100 of a patient 102 and a portion of the spinal cord 104. The pudendal nerves 108 exit the sacral cord and branch into the dorsal penile/clitoral nerves 110. Fig. 1 further illustrates Alcock's Canal 112 and the pubic symphysis 114. In the illustration of Fig. 1, a cuff electrode 116 is placed around the left dorsal penile/clitoral nerve 110. The electrode 116 is connected to an implanted pulse generator 118 via an implanted lead 120. The pulse generator 118 comprises a battery (not shown in Fig. 1) and is configured to convey pulses of electrical current through the electrode 116 via the lead 120, so as to provide electrical stimulation to the dorsal penile/clitoral nerve 110. The battery may be chargeable by means of inductive wireless power transmission providing electromagnetic signals at a frequency of below 315 kHz.

An external, wrist worn device 122 is provided for wirelessly communicating with the pulse generator 118. In one embodiment of the invention, the wrist worn device 122 represents a triggering mechanism for causing the pulse generator 118 to pass a series of electrical pulses to the electrode. Alternatively, or additionally, the wrist worn device 112 may be configured as a programming device for setting stimulation parameters or for otherwise programming or configuring the implanted pulse generator 118. The pulse generator 118 may be configured for providing data to the external device 122, such as e.g. a low battery warning of the pulse generator 118.

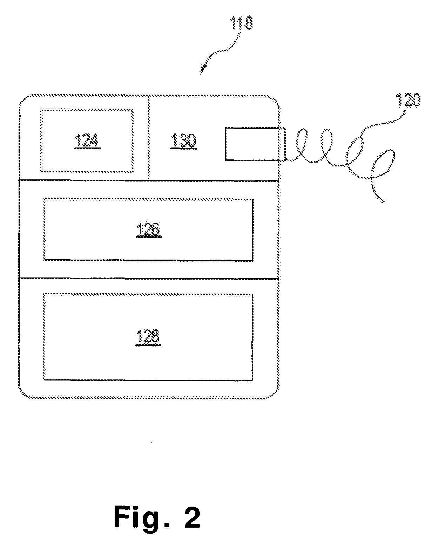

Fig. 2 generally shows the main components of the pulse generator 118. The pulse generator includes a telemetry unit 124 for receiving and/or sending information to/from an external device, such as the wrist worn device 122 shown in Fig. 1. Further, the pulse generator includes a pulse generating circuitry 126, and a battery 128. The battery 128 is preferably hermetically sealed within a housing of the pulse generator 118. The pulse generator contains a connector 130 to attach the implantable lead 120.

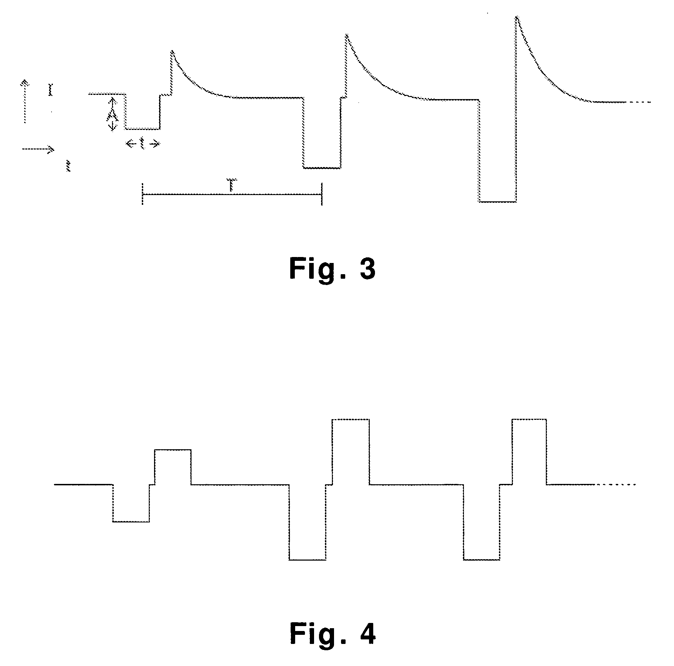

Figs. 3 and 4 are graphical representations of biphasic stimulus current outputs from the pulse generator during initial stepwise increase of the pulse charges upon activation of the pulse generator. In the example shown, the current amplitudes A of the pulse are stepwise increased. It should however be understood that the pulse charges may also be increased by extending the pulse width t, shortening the intervals between the pulses T, or stepwise increase the pulse voltages. The chart of Fig. 3 illustrates de-charge of a series capacitor upon short-circuiting of electrodes. In the example of Fig. 4, alternating polarity is achieved by alternating the current through the electrode by means of hardware or software control embedded in the pulse generator.

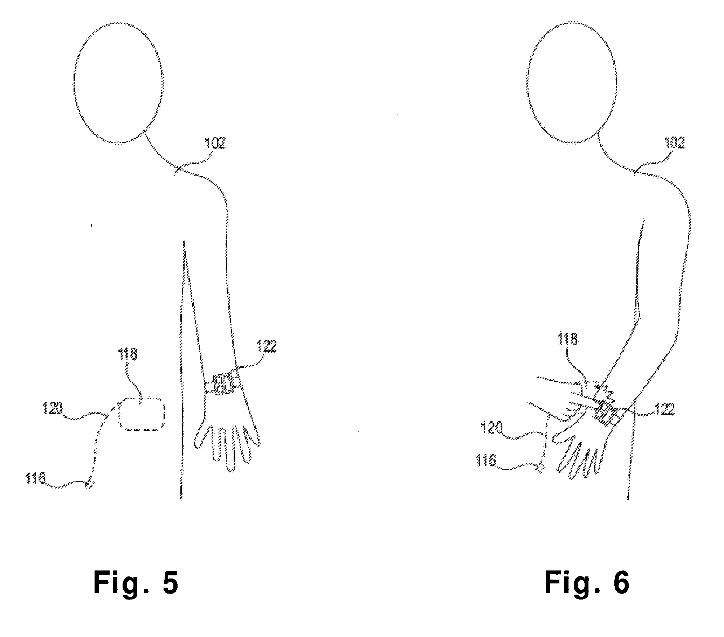

As mentioned above, the wrist worn device 122 may serve as a wireless external triggering or programming device for programming or configuring the pulse generator. As shown in Figs. 5 and 6, the patient 102 may trigger or program the pulse generator 118 by locating the wrist worn device 122 in the vicinity of the implanted pulse generator 118 and activating a programming action, so as to wirelessly transmit an appropriate triggering signal, programming or configuration data to the pulse generator 118, as shown in Fig. 6.

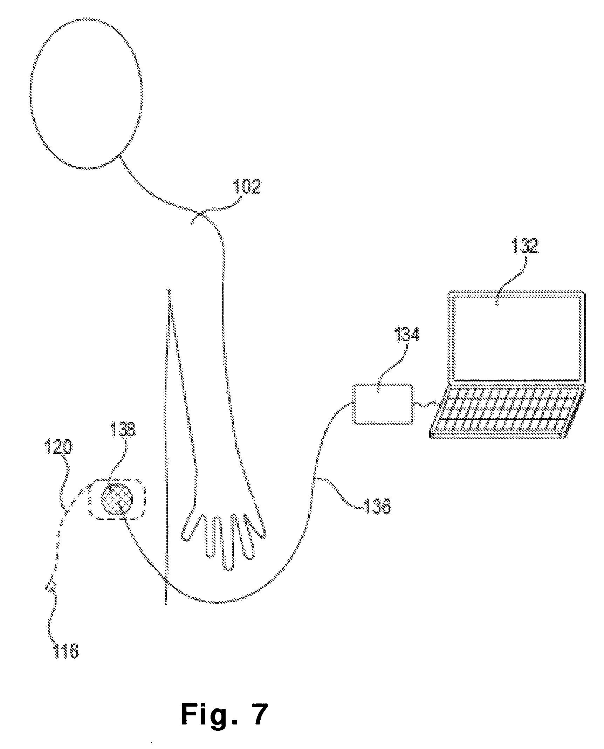

Fig. 7 illustrates a clinical programming system for a system according to the invention. The programming system includes a personal computer 132, which is typically operated by a physician, and a programming box 134. The programming box is connected to an inductive coil 138 via a lead 136. Programming of the implanted pulse generator 118 is performed via wireless, inductive telemetry from the inductive coil through the skin of the patient.

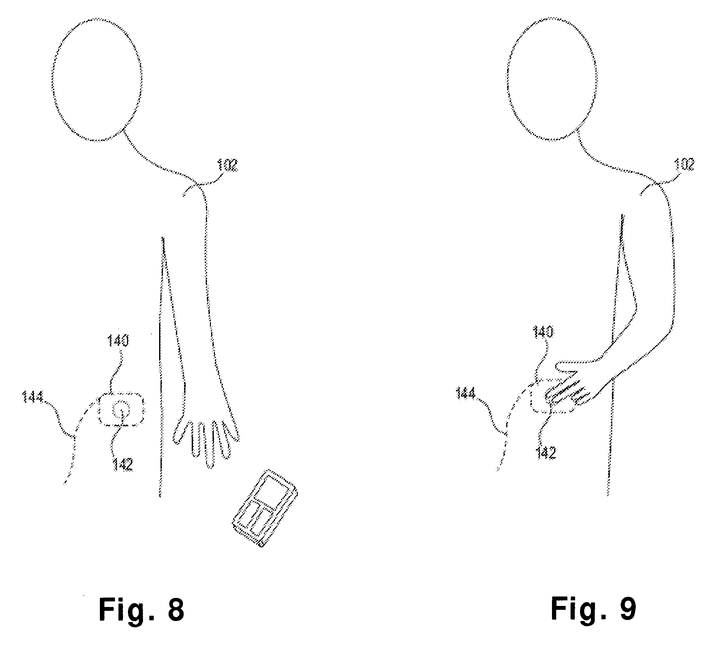

Figs. 8 and 9 are illustrations of an implanted triggering mechanism 140. The triggering mechanism is activatable by mechanical impact to an outer surface portion of the patient's body, e.g. by a push button 142, which is activatable by application of a finger pressure to the outer surface portion of the body, as illustrated in Fig. 9. The triggering mechanism 140 may also house the pulse generator, in which case a distal end of the lead 144 is connected to the electrode (not shown). Alternatively, the pulse generator may be provided as a separately implanted component, whereby the lead 144 interconnects the triggering mechanism and the pulse generator. An external programming device 146 is provided for programming or configuring the implanted pulse generator and/or the implanted triggering mechanism 140 via wireless data transmission.

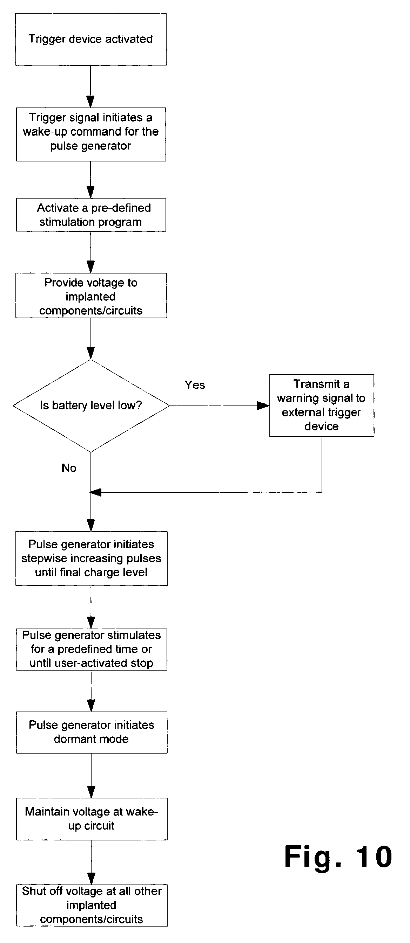

Fig. 10 shows a flowchart of an embodiment of a process occurring in the systems described above. Initially, the pulse generator is in a dormant mode, in which no voltage is maintained at any of the implanted components and circuits, except at the wake-up circuit of the pulse generator. When the patient activates the trigger device (triggering mechanism) 122 or 140, a trigger signal is provided to initiate a wake-up command for the pulse generator 118 via its wake-up circuit. Further, a voltage is applied to the implanted components and circuits, at which no voltage was maintained in the dormant mode. The wake-up command causes the pulse generator to enter an active mode and to activate a predefined stimulation program. If the pulse generator detects a low battery status, a wireless warning signal is provided to an external device, such as the external triggering device 122 or an external programming device. Subsequently, the pulse generator initiates a stepwise increase of pulse charges from zero up to a final charge level, and pulse stimuli are provided for a predetermined period of time, or until the patient deactivates the pulses. Finally, the pulse generator enters a dormant mode, and voltage is cut off to the implanted components and circuits, expect for the wake-up circuit of the pulse generator, at which a voltage is maintained.

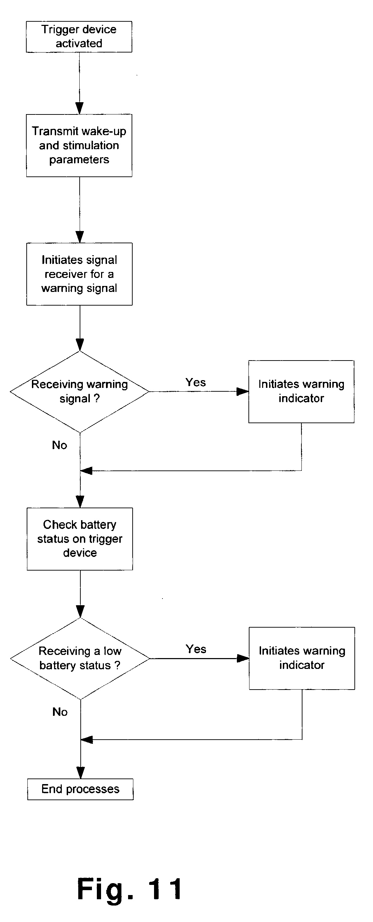

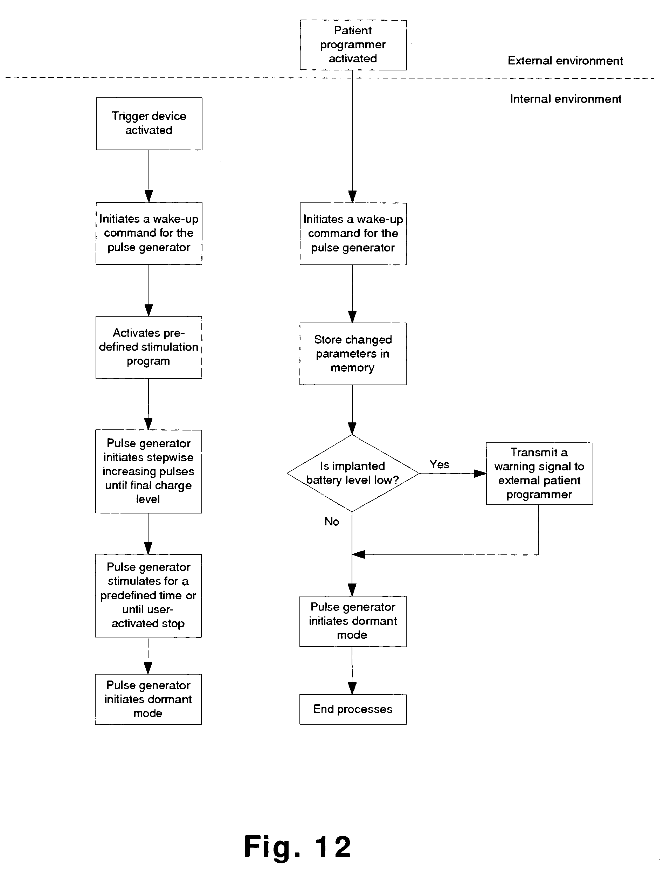

Fig. 11 illustrates a similar embodiment of a process occurring in the external parts of the systems described above. When the patient activates the trigger device (triggering mechanism 122 or user activateable device 162, see Fig. 12), a wake-up command is sent to the wake-up circuit of the pulse generator (directly from triggering mechanism 122 or via the skin-borne device 160, see the below description of Fig. 12), and certain stimulation parameters are possibly also sent to the pulse generator. An external signal receiver for a warning signal is initiated, and if a warning signal indicative of e.g. a low battery status of the pulse generator, is received, a warning indicator is initiated. In the same manner, a warning indicator may be initiated, if there is a low battery warning from the trigger device.

Fig. 12 shows a flow chart of a process occurring the system of Figs. 8 and 9, relying on a triggering mechanism, which is activatable upon application of a mechanical impact to an outer surface portion of the patient's body.

In the left part of Fig. 12, the pulse generator is initially in a dormant mode. When the patient activates the trigger device (triggering mechanism) 140, a trigger signal is provided to initiate a wake-up command for the pulse generator. The wake-up command causes the pulse generator to enter an active mode and to activate a predefined stimulation program. The pulse generator then initiates a stepwise increase of pulse charges from zero up to a final charge level, and pulse stimuli are provided for a predetermined period of time, or until the patient deactivates the pulses. Finally, the pulse generator enters a dormant mode.

The right part of Fig. 12 illustrates a programming process for the system of Figs. 8 and 9. When a patient or physician programmer is activated, a wake-up command for the pulse generator is initiated. Changed parameters are then stored in a memory of the pulse generator, and a warning signal is transmitted to the external patient programmer if a low battery level is detected.

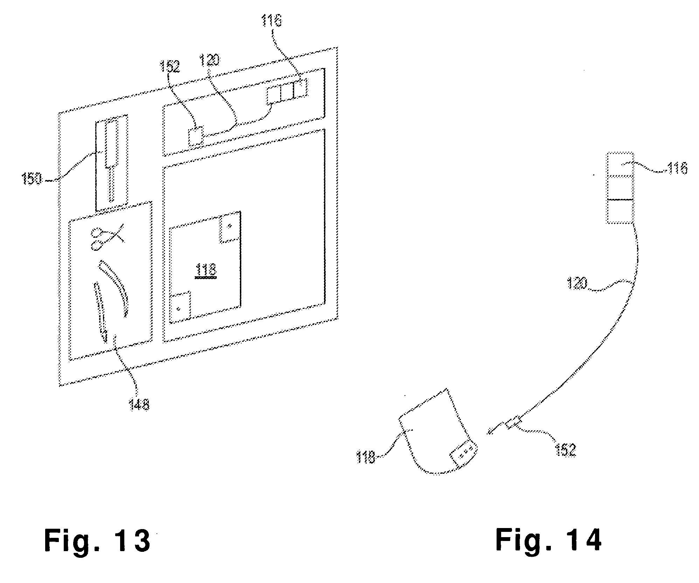

Fig. 13 illustrates an embodiment of a sterilized surgical kit. The kit includes several sterilized packages. One package contains the pulse generator 118. Another package contains the electrode 116, connected to a connector 152 via the lead 120. Further packages 148 and 150 include surgical tools for implanting the electrode 116, pulse generator 118 and lead 120. The surgical tools may for example include tunneling tools for tunneling the lead and/or electrode through the patient's body during surgical intervention. As shown in Fig. 14, the connector 152 allows the lead 120 to be releasably connected to the pulse generator 118.

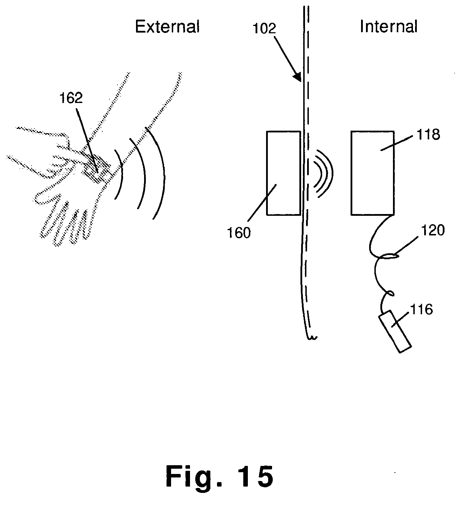

In the embodiment of Fig. 15, the user-operable triggering mechanism comprises a skin-borne device 160 releasbly attached to an abdominal wall of the user in the vicinity of the implanted pulse generator 118 and electrode 116. The user-operable triggering mechanism further comprises a hand-held user activatable device 162 for transmitting radio frequency control signals to the skin-borne device 160, which in turn generates inductive wireless signals to the pulse generator 118.

## Claims

1. A system for the treatment of urge incontinence by electrical stimulation of pudendal nerve afferents of a living being, including:
- at least one implantable electrode (116) configured to be placed on, at, in, around or near a portion of a pudendal nerve (108) or its branches (110);
- a pulse generator (118) configured to:
- provide a sequence of electrical pulses to the at least one electrode (116) in order to achieve said electrical stimulation of the pudendal nerve afferents;
- enter an active mode when a control signal is provided to the pulse generator (118), the pulse generator (118) being configured to provide said sequence of pulses in the active mode;
- enter a dormant mode when the sequence of electrical pulses has been completed, **characterised by**:
- a wake-up circuit for providing said control signal to the pulse generator (118) to cause it to exit the dormant mode and enter the active mode;
- at least one power source (128) for powering the pulse generator (118), wherein the power source (128) is configured such that no voltage is maintained at the pulse generator in the dormant mode;
- the pulse generator (118) being configured to receive control signals transmitted via wireless inductive telemetry; and
- an external, non-implantable device (122; 160) for transmitting energy to the wake-up circuit by wireless inductive energy transfer for initiating wake-up of the pulse generator.

2. A system according to claim 1, wherein the wake-up circuit is entirely housed or integrated in the pulse generator (118).

3. A system according to claim 1 or 2, wherein the pulse generator is further configured to, subsequent to having entered the active mode, stepwise increase charges of electrical pulses from an initial level to a final level, and to subsequently provide the electrical pulses at said final level for a predetermined period of time.

4. A system according to any of the preceding claims, wherein the pulse generator (118) is configured to control the amplitude of current of the electrical stimulation pulses.

5. A system according to any of the preceding claims, wherein the pulse generator (118) is configured to stepwise increase the pulse charges from the initial level to the final level during a period of time of about 0.1 seconds to about 40 seconds.

6. A system according to any of the preceding claims, wherein the pulse generator (118) is configured to provide the pulses at a frequency of about 5 Hz to about 50 Hz.

7. A system according to any of the preceding claims, wherein the initial level provides no current, and wherein the final level provides charges at a current from about 0.01 mA to about 100 mA.

8. A system according to any of the preceding claims, wherein the at least one electrode (116) comprises an electrode configured to be placed at, on, in, near or around a nerve (108; 110) or nerve branch containing pudendal nerve afferents.

9. A system according to any of the preceding claims, wherein the pulse generator (118) is configured to vary the widths of said electrical pulses at least when stepwise increasing the pulse amplitudes from the initial level to the final level.

10. A system according to any of the preceding claims, wherein the pulses are provided at a pulse width of about 50 µs to about 400 µs.

11. A system according to any of the preceding claims, wherein the at least one electrode (116) is implantable at a left and/or right branch of the dorsal penile nerve (110) at or near the dorsum penis or the base of the clitoris.

12. A system according to any of the preceding claims, wherein said control signal comprises an inductive wireless signal at a frequency below 315 kHz.

13. A system according to any of the preceding claims, further comprising a user-operable triggering mechanism (122;140) configured to transmit said control signal to the implantable pulse generator and/or to the wake-up circuit in order to trigger the sequence of electrical pulses.

14. A system according to claim 13, wherein the triggering mechanism (122; 140) comprises a user-interface configured to allow the user to set stimulation parameters.

15. A system according to claim 13 or 14, wherein the user-operable triggering mechanism (122) comprises:
- a skin-borne device (160) for transmitting inductive wireless control signal to the implantable pulse generator (118), and
- a user activatable device (162) for transmitting a wireless radio frequency signal to the skin-borne device (160) upon user activation of the user activatable device;
the skin-borne device (160) being configured to receive the wireless radio frequency signal transmitted from the user activatable device (162) and to generate the inductive wireless control signal for the implantable pulse generator (118) in response thereto.

## Patentansprüche

1. System zur Behandlung von Dranginkontinenz durch elektrische Stimulation von afferenten Schamnerven eines Lebewesens, enthaltend:
- mindestens eine implantierbare Elektrode (116), die zum Anordnen an, bei, in, um oder nahe einem bzw. einen Teil eines Schamnervs (108) oder seiner Verzweigungen (110) konfiguriert ist;
- einen Pulsgenerator (118), der konfiguriert ist zum:
- Bereitstellen einer Abfolge elektrischer Pulse an der mindestens einen Elektrode (116), um die elektrische Stimulation der afferenten Schamnerven zu erreichen;
- Wechseln in einen Aktivmodus, wenn dem Pulsgenerator (118) ein Steuersignal bereitgestellt wird, wobei der Pulsgenerator (118) zum Bereitstellen der Abfolge von Pulsen im Aktivmodus konfiguriert ist;
- Wechseln in einen Ruhemodus, wenn die Abfolge von Pulsen beendet ist,
**gekennzeichnet durch**:
- eine Weckschaltung zum Bereitstellen des Steuersignals beim Pulsgenerator (118), um diesen zu veranlassen, den Ruhemodus zu verlassen und in den Aktivmodus zu wechseln;
- mindestens eine Leistungsquelle (128) zur Leistungsversorgung des Pulsgenerators (118), wobei die Leistungsquelle (128) so konfiguriert ist, dass im Ruhemodus keine Spannung am Pulsgenerator aufrechterhalten wird;
- der Pulsgenerator (118) zum Empfangen von Steuersignalen konfiguriert ist, die **durch** drahtlose induktive Telemetrie übertragen werden; und
- eine externe, nicht implantierbare Vorrichtung (122; 160) zum Übertragen von Energie zur Weckschaltung **durch** drahtlose induktive Energieübertragung, um ein Wecken des Pulsgenerators einzuleiten.

2. System nach Anspruch 1, wobei die Weckschaltung vollständig im Pulsgenerator (118) aufgenommen oder integriert ist.

3. System nach Anspruch 1 oder 2, wobei der Pulsgenerator, nachdem er in den Aktivmodus gewechselt hat, zum stufenweisen Erhöhen von Ladungen elektrischer Pulse von einem anfänglichen Pegel auf einen endgültigen Pegel und zum anschließenden Bereitstellen der elektrischen Pulse bei dem endgültigen Pegel für eine vorbestimmte Zeitperiode konfiguriert ist.

4. System nach einem der vorangehenden Ansprüche, wobei der Pulsgenerator (118) zum Steuern der Stromamplitude der elektrische Stimulationspulse konfiguriert ist.

5. System nach einem der vorangehenden Ansprüche, wobei der Pulsgenerator (118) zum schrittweisen Erhöhen der Pulsladungen vom anfänglichen Pegel auf den endgültigen Pegel für eine vorbestimmte Zeitperiode von etwa 0,1 Sekunden bis etwa 40 Sekunden konfiguriert ist.

6. System nach einem der vorangehenden Ansprüche, wobei der Pulsgenerator (118) zum Bereitstellen der Pulse bei einer Frequenz von etwa 5 Hz bis etwa 50 Hz konfiguriert ist.

7. System nach einem der vorangehenden Ansprüche, wobei der anfängliche Pegel keinen Strom liefert und wobei der endgültige Pegel Ladungen bei einem Strom von etwa 0,01 mA bis etwa 100 mA liefert.

8. System nach einem der vorangehenden Ansprüche, wobei die mindestens eine Elektrode (116) eine Elektrode umfasst, die zum Anordnen bei, an, in, nahe oder um einem bzw. einen Nerv (108; 110) oder Nervenzweig, enthaltend afferente Schamnerven, konfiguriert ist.

9. System nach einem der vorangehenden Ansprüche, wobei der Pulsgenerator (118) zum Variieren der Breiten der elektrischen Pulse zumindest beim stufenweisen Erhöhen der Pulsamplituden vom anfänglichen Pegel auf den endgültigen Pegel konfiguriert ist.

10. System nach einem der vorangehenden Ansprüche, wobei die Pulse bei einer Pulsbreite von etwa 50 µs bis etwa 400 µs bereitgestellt werden.

11. System nach einem der vorangehenden Ansprüche, wobei die mindestens eine Elektrode (116) an einem linken und/oder rechten Zweig des dorsalen Penisnervs (110) an oder nahe dem Dorsum Penis oder der Basis der Klitoris implantierbar ist.

12. System nach einem der vorangehenden Ansprüche, wobei das Steuersignal ein induktives drahtloses Signal bei einer Frequenz unter 315 kHz umfasst.

13. System nach einem der vorangehenden Ansprüche, des Weiteren umfassend einen vom Benutzer betätigbaren Auslösemechanismus (122, 140), der zum Übertragen des Steuersignals zu dem implantierbaren Generator und/oder der Weckschaltung konfiguriert ist, um die Abfolge von elektrischen Pulsen auszulösen.

14. System nach Anspruch 13, wobei der Auslösemechanismus (122; 140) eine Benutzerschnittstelle umfasst, die dazu ausgebildet ist, dem Benutzer die Einstellung von Stimulationsparametern zu ermöglichen.

15. System nach Anspruch 13 oder 14, wobei der vom Benutzer betätigbare Auslösemechanismus (122) umfasst:
- eine auf der Haut getragene Vorrichtung (160) zum Übertragen des induktiven drahtlosen Steuersignals zum implantierbaren Pulsgenerator (118) und
- eine vom Benutzer aktivierbare Vorrichtung (162) zum Übertragen eines drahtlosen Funkfrequenzsignals der auf der Haut getragenen Vorrichtung (160) nach Aktivierung der vom Benutzer aktivierbaren Vorrichtung (162) durch den Benutzer;
wobei die auf der Haut getragene Vorrichtung (160) zum Empfangen des drahtlosen Funkfrequenzsignals konfiguriert ist, das von der vom Benutzer aktivierbaren Vorrichtung (162) übertragen wird, und als Reaktion darauf zum Erzeugen des induktiven drahtlosen Steuersignals für den implantierbaren Pulsgenerator (118).

## Revendications

1. Système de traitement d'une incontinence impérieuse par stimulation électrique des afférences du nerf pudendal d'un être vivant, y compris :
- au moins une électrode implantable (116) configure pour être placée sur, dans, autour ou près d'une partie du nerf pudendal (108) ou de ses ramifications (110) ;
- un générateur d'impulsions (118) configuré pour :
- fournir une séquence d'impulsions électriques à l'au moins une électrode (116) afin d'obtenir ladite stimulation électrique des afférences du nerf pudendal ;
- se mettre en mode actif lorsque le signal de commande est fourni au générateur d'impulsions (118), le générateur d'impulsions (118) étant configuré pour fournir ladite séquence d'impulsions en mode actif ;
- se mettre en mode inactif lorsque la séquence d'impulsions électriques a été obtenue, **caractérisé par** :
- un circuit d'activation pour fournir audit signal de commande au générateur d'impulsions (118) pour le faire passer du mode inactif au mode actif ;
- au moins une source d'alimentation (128) pour alimenter le générateur d'impulsions (118), dans lequel la source d'alimentation (128) est configurée de sorte qu'aucune tension n'est maintenue au niveau du générateur d'impulsions en mode inactif ;
- le générateur d'impulsions (118) étant configuré pour recevoir des signaux de commande transmis par télémétrie inductive sans fil, et
- un dispositif non implantable externe (122, 160) pour transmettre de l'énergie au circuit d'activation par transfert d'énergie par induction sans fil pour initier l'activation du générateur d'impulsions.

2. Système selon la revendication 1, dans lequel le circuit d'activation est entièrement hébergé ou intégré dans le générateur d'impulsions (118).

3. Système selon la revendication 1 ou 2, dans lequel le générateur d'impulsions est en outre configuré pour, après s'être mis en mode actif, augmenter progressivement les charges des impulsions électriques d'un niveau initial à un niveau final, et fournir ensuite des impulsions électriques audit niveau final, pendant une période de temps prédéterminée.

4. Système selon l'une quelconque des revendications précédentes, dans lequel le générateur d'impulsions (118) est configuré pour contrôler l'amplitude du courant des impulsions obtenues par stimulations électriques.

5. Système selon l'une quelconque des revendications précédentes, dans lequel le générateur d'impulsions (118) est configuré pour augmenter progressivement les charges des impulsions électriques d'un niveau initial à un niveau final pendant une période de temps d'environ 0,1 secondes à environ 40 secondes.

6. Système selon l'une quelconque des revendications précédentes, dans lequel le générateur d'impulsions (118) est configuré pour fournir des impulsions à une fréquence d'environ 5 Hz à environ 50 Hz.

7. Système selon l'une quelconque des revendications précédentes, dans lequel le niveau initial ne fournit pas de courant, et dans lequel le niveau final fournit des charges à une intensité de courant d'environ 0,01 mA à environ 100 mA.

8. Système selon l'une quelconque des revendications précédentes, dans lequel l'au moins une électrode (116) comprend une électrode configurée pour être placée sur, dans, près ou autour d'un nerf (108; 110) ou d'une ramification contenant les afférences du nerf pudendal.

9. Système selon l'une quelconque des revendications précédentes, dans lequel le générateur d'impulsions (118) est configuré pour faire varier les largeurs desdites impulsions électriques de manière progressive, au moins lors de l'augmentation des amplitudes des impulsions du niveau initial au niveau final.

10. Système selon l'une quelconque des revendications précédentes, dans lequel les impulsions sont fournies à des largeurs d'environ 50 ps à environ 400 ps.

11. Système selon l'une quelconque des revendications précédentes, dans lequel l'au moins une électrode (116) est implantable sur une ramification gauche et/ou droite du nerf dorsal du pénis (110) au niveau ou près de la face dorsale du pénis ou de la base du clitoris.

12. Système selon l'une quelconque des revendications précédentes, dans lequel le ledit signal de commande comprend un signal inductif sans fil à une fréquence inférieure à 315 kHz.

13. Système selon l'une quelconque des revendications précédentes, comprenant en outre un mécanisme de déclenchement actionnable par l'utilisateur (122, 140) configuré pour transmettre ledit signal de commande au générateur d'impulsions implantable et/ou au circuit d'activation pour déclencher la séquence de impulsions électriques.

14. Système selon la revendication 13, dans lequel le mécanisme de déclenchement (122; 140) comprend une interface utilisateur configurée pour permettre à l'utilisateur de régler les paramètres de stimulation.

15. Système selon la revendication 13 ou 14, dans lequel le mécanisme de déclenchement actionnable par l'utilisateur (122) comprend :
- un dispositif relié à la peau (160) pour transmettre des signaux de commande inductifs sans fil au générateur d'impulsions implantable (118), et
- un dispositif actionnable par l'utilisateur (162) pour transmettre un signal de fréquence radio sans fil au dispositif relié à la peau (160) lors de l'activation par l'utilisateur du dispositif pouvant être activé par l'utilisateur, le dispositif relié à la peau (160) étant configuré pour recevoir le signal de fréquence radio sans fil transmis par le dispositif activable par l'utilisateur (162) et pour générer le signal de commande inductif sans fil vers le générateur d'impulsions implantable (118) en réponse à celui-ci.
